# EUROPEAN PATENT APPLICATION

(11) **EP 4 019 030 A1**
(43) Date of publication of application: **29.06.2022**
(21) Application number: 20217233.4
(22) Date of filing: 24.12.2020
(51) Int. Cl.: A61K 36/185, B01D 1/00

(54) **METHOD AND APPARATUS TO INFUSE DRIED CANNABIS SATIVA**

(71) Applicant: Cannterp Inc., Acton, ON L7J 2L8 (CA)
(72) Inventor: GREB, Kathleen, Acton, Ontario L7J 2L8 (CA)
(74) Representative: Hancox, Jonathan Christopher

(57) **Abstract**

The present invention relates to a method of infusing dried cannabis or hemp flower with terpenes and an apparatus used to infuse dried cannabis or hemp flower with terpenes comprising an openwork shell, an absorbent material and a liquid.

## Description

### FIELD OF THE INVENTION

This invention generally relates to an improved method and apparatus to infuse dried cannabis and hemp flower with essential oils in the form of terpenes.

### DESCRIPTION OF THE PRIOR ART

In order to be consumed, cannabis sativa must first go through a drying process. However, when the cannabis sativa flower goes through the drying process it can lose up to 90% of its terpenes. Re-infusing the dried cannabis flower with lost terpenes or terpenoids prior to consumption to achieve intended effects is desirable. Terpenes are aromatic oils that color cannabis varieties with distinctive flavors like citrus, berry, mint, and pine. Terpenes play a key role in differentiating the effects of various cannabis strains. There are over 100 different identified terpenes in the cannabis plant, and while the differences can be subtle, much progress has been made in making classification of terpenes and their effects easy for patients and consumers to understand. Broadly, terpenes can be broken down into sweet, sour, spicy, or bitter - with each category further breaking down into more specific smells. These specific smells in turn correlate to the effects of that plant. The known methods of re-infusing dried cannabis flower with terpenes typically have the terpenes in direct contact with the dried cannabis flower resulting in over-saturating the dried cannabis flower, creating a sticky, oily mess which is undesirable to consumers and inhibits the effects of the consumed cannabis.

### SUMMARY OF THE INVENTION

The present disclosure provides a method to infuse dried cannabis and hemp flower with essential oils in the form of terpenes to improve humidity, taste and effect. The present method provides placing a perforated casing containing an absorbent material with the essential oil added to the absorbent material into a container, adding dried cannabis and hemp flower and closing the container. In a preferred embodiment the invention is used to infuse dried cannabis and hemp flower with terpenes that may add additional scent, taste, moisture and possible effects to the dried cannabis and hemp flower. The present invention allows the consumer to choose which terpenes to use for the re-infusion providing a selection and range of desired effects that the dried cannabis or hemp flower will produce upon consumption.

In another embodiment the present invention provides an apparatus to be used for infusing dried cannabis and hemp flower with essential oils, comprising a casing formed of a material inert or impervious to the essential oils and having one or more apertures and an absorbent material contained within the casing. The absorbent material is capable of retaining and diffusing the essential oils applied to the absorbent material. The preferred absorbent material is a sponge sized and shaped to fit loosely within the casing.

Further features of the invention will be described or will become apparent in the course of the following detailed description.

### BRIEF DESCRIPTION OF THE DRAWINGS

In order that the invention may be more clearly understood, the preferred embodiment thereof will now be described in detail by way of example, with reference to the accompanying drawings, in which:
Figure 1 is a top plan view of one embodiment of an openwork shell according to the present invention that is inside a container along with dried cannabis or hemp flower;
Figure 2 is a schematic perspective drawing of one embodiment of an openwork shell according to the present invention that is closed and encases an absorbent material;
Figure 3 is an assembly drawing of the openwork shell and absorbent material according to Figure 2;
Figure 4 is a side plan view of one half of the openwork shell shown in Figure 2;
Figure 5 is a schematic perspective drawing of one half of the openwork shell shown in Figure 4;
Figure 6 is a side plan view of one half of the openwork shell and the absorbent material shown in Figure 2.

Similar references are used in different figures to denote similar components.

### DETAILED DESCRIPTION OF THE PREFERRED EMBODIMENT

In this disclosure, the following terms have the following meanings:

"Absorbent material": a material having capacity to absorb liquids and permit them to evaporate and diffuse under normal conditions.

"Cannabis Sativa" means any species of the Cannabaceae family that includes both marijuana and hemp varieties.

"Dried cannabis or hemp flower": dried pieces of the cannabis or hemp plant consisting of flower buds and leaves that have been cured and dried and ready to smoke or ingest in edibles.

"Casing with one or more apertures": an outer form or case with a hollow interior and consisting of any material inert or impervious to absorption of liquids used in the present invention and having one or more openings or holes through its outer surface and in communication to the casing interior for example an openwork shell or cage like structure.

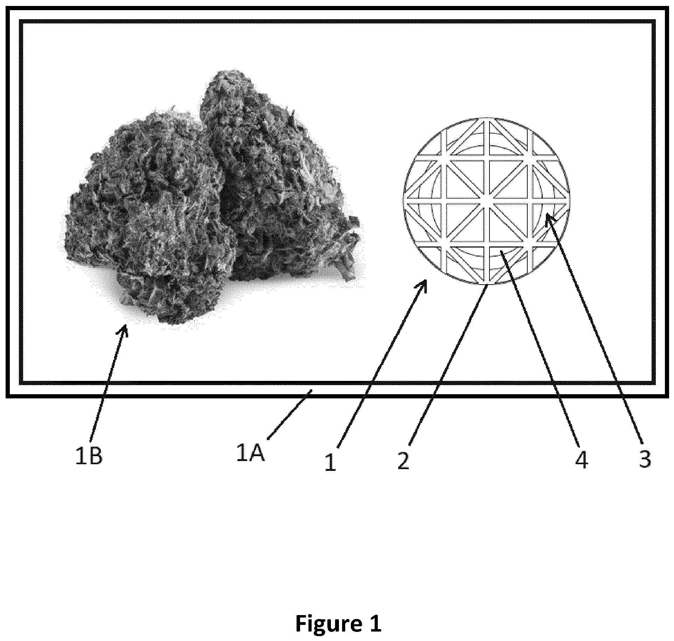

Referring to Figure 1, the present disclosure provides a method of infusing dried cannabis and hemp flower with a liquid preferably an essential oil including terpenes or terpenoids to improve humidity, taste and effect. The method comprises placing an absorbent material 4 such as a sponge inside a casing with one or more apertures such as an openwork shell 2. The absorbent material 4 is preferably a sponge but can also be another material capable of absorbing and diffusing essential oils such as felt discs, etc. The selected liquid preferably containing terpenes or terpenoids can be applied to the absorbent material 4 before or after placing the absorbent material 4 into the casing or openwork shell 2. The liquid applicator means for applying the liquid to the absorbent material 4 can be selected from a syringe or possibly an eye dropper. Alternatively, the liquid may be applied to the absorbent material 4 by soaking the absorbent material in the liquid, removing the absorbent material from the liquid, squeezing the excess liquid from the absorbent material, and letting the absorbent material with the liquid applied sit for a sufficient amount of time to absorb the liquid and fully saturate. In the preferred embodiment, a syringe is used to apply 0.5 ml of liquid to the sponge 4. The amount of liquid added to the sponge is illustrative but not restrictive as it depends on the surface area of the sponge, the amount of cannabis or hemp being infused and the size of the sponge. In the preferred embodiment, the openwork shell 2 encasing the absorbent material 4 with the liquid applied is preferably placed inside a container 1A wherein the container 1A preferably holds dried cannabis or hemp flower 1B. The apparatus 1 can be sequentially used to infuse a plurality of batches of dried cannabis or hemp flower. To continue infusing the dried cannabis or hemp flower or to infuse subsequent batches of cannabis or hemp flower, additional liquid may be applied to the absorbent material 4 or a new absorbent material with the liquid applied may be used. In the preferred embodiment, both the absorbent material 4 and the dried cannabis or hemp flower 1B are not over saturated by the liquid.

The selected liquid may include essential oils or one of the many different identified terpenes normally found in the cannabis or hemp plant such as limonene (having a citrus aroma, known for its stress-relieving and mood-enhancing effects), pinene (having a pine, woody aroma, known to boost energy, improve memory and alertness, acts as bronchodilator, anti-inflammatory and local antiseptic), myrcene (has an earthy aroma, known to have sedating and relaxing effects) and linalool (has a light floral aroma, known to reduce stress, anti-anxiety, antidepressant, and act as a sedative) among a few.

The casing or openwork shell 2 containing the absorbent material 4 with the applied liquid is placed into a container 1A. The absorbent material 4 can be selected from a sponge or another material capable of absorbing and diffusing a liquid, preferably essential oils, such as sponge, felt discs, etc. In the preferred embodiment, the absorbent material 4 is a sponge that provides sufficient surface area to enable evaporation of the essential oil at the rate required to properly infuse the dried cannabis or hemp flower 1B. The absorbent material 4 is sufficiently saturated by the liquid to infuse the dried cannabis or hemp flower 1B with the desired level of humidity, taste or effect and to prevent direct contact with the dried cannabis or hemp flower 1B which will result in over-saturating the dried cannabis or hemp flower 1B and creating a sticky, oily mess. In the embodiment illustrated, the casing or openwork shell 2 encasing the absorbent material 4 is placed inside a container 1A containing dried cannabis or hemp flower 1B where it is enclosed in a controlled environment separated from the outside environment and the liquid can evaporate and infuse the dried cannabis or hemp flower 1B. As noted earlier the liquid can be applied before or after the absorbent material 4 is placed in the openwork shell 2. The container 1A can be selected from a sealable plastic bag, a jar, a tin can, or a humidor. Any number of openwork shells containing the absorbent material 4 with the liquid applied may be placed inside the sealed container 1A to sufficiently infuse larger amounts of stored dried cannabis or hemp flower. In the preferred embodiment, one openwork shell is added into the container for every 3.5 grams of dried cannabis or hemp flower. The number of openwork shells added to the container is illustrative but not restrictive as the number of openwork shells added depends on the surface area of the absorbent material, the amount of cannabis or hemp being infused and the size of the absorbent material. Once the openwork shell containing the absorbent material with the liquid applied is added into the container with dried cannabis or hemp, the liquid will immediately begin to infuse the cannabis or hemp. In the preferred embodiment, the openwork shell containing the absorbent material with the liquid applied is left in the container with the cannabis or hemp for a minimum of 24 hours before the cannabis or hemp is consumed and will continue to infuse for 1 to 3 months from time of first infusion.

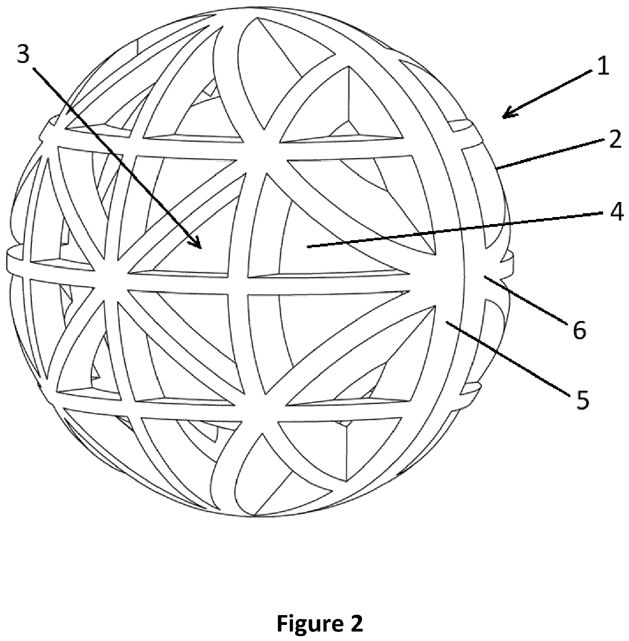

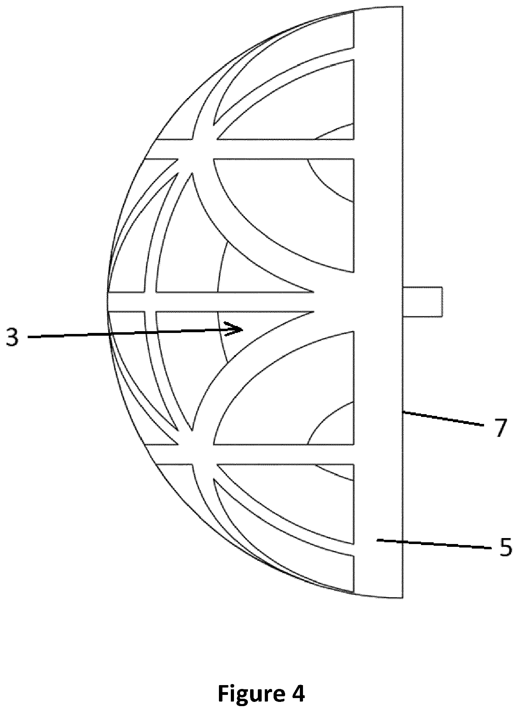

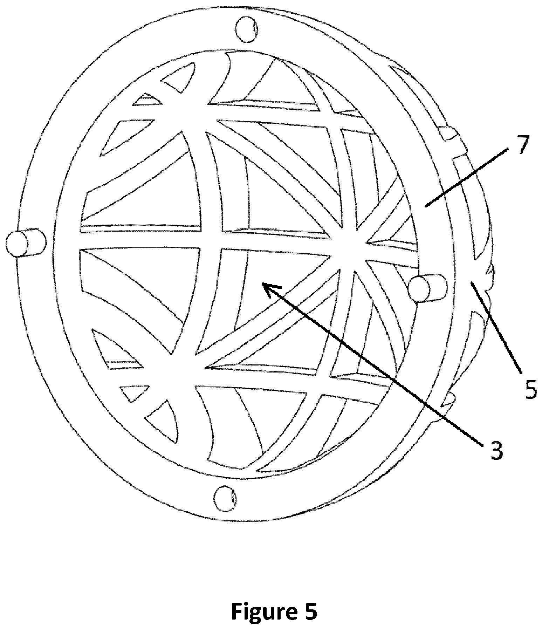

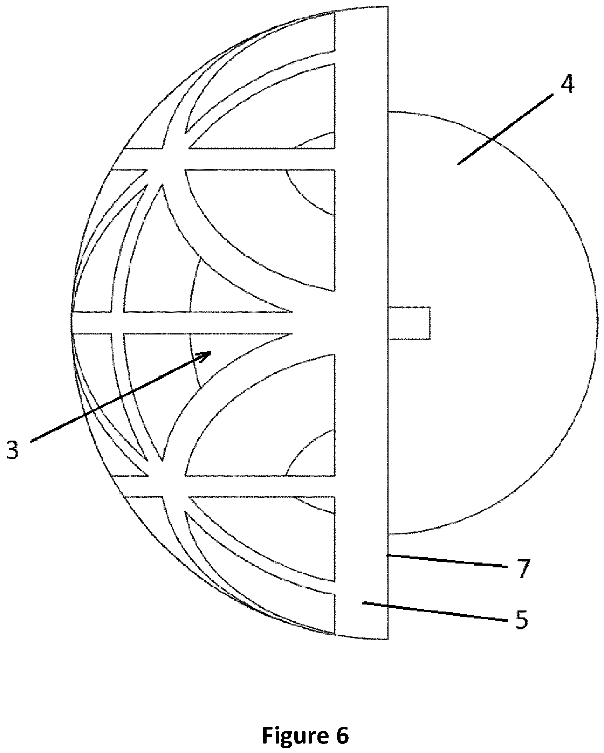

Referring to Figures 1 to 6, the present disclosure provides an apparatus used in association with the method above generally indicated at 1. The apparatus 1 according to the present invention to be used for infusing dried cannabis or hemp flower 1B with a liquid, in one embodiment comprises a casing 2 formed of a material inert or impervious to the liquid and having one or more apertures and an absorbent material 4 contained within the casing 2. The absorbent material 4 is capable of retaining and diffusing the liquid, preferably essential oils or one of the many different identified terpenes normally found in the cannabis or hemp plant, applied to the absorbent material 4. The preferred absorbent material 4 is a sponge, sized and shaped to fit loosely within the casing 2.

In the embodiment illustrated in Figures 1-6, the casing is an openwork shell 2 that is spherical in shape and has multiple apertures covering the entire outer surface of the openwork shell. An aperture is generally indicated at 3. The openwork shell 2 contains an absorbent material 4. The apertures communicate with the interior of the openwork shell 2 and are sized and shaped so as to not allow direct contact between the absorbent material 3 and the dried cannabis or hemp flower 1B and so as to allow the dried cannabis or hemp flower 1B to be sufficiently infused. In the preferred embodiment, the apertures are triangular in shape. While in the embodiment illustrated the openwork shell 2 has a spherical shape it may take any shape or form so long as it keeps the cannabis or hemp flower out of direct contact with the liquid and has sufficient aperture area and size to allow the liquid to evaporate and sufficiently infuse the dried cannabis or hemp flower 1B. The openwork shell 2 may consist of a flexible or rigid material. In the embodiment illustrated in Figures 1-6, the openwork shell 2 consists of a rigid material. The openwork shell 2 contains an absorbent material 4 of an appropriate shape and size to fit loosely inside the closed openwork shell 2 and which is used to hold the liquid prior to infusing the dried cannabis or hemp flower 1B. Additionally, the openwork shell 2 has adequate thickness to encase the absorbent material 4 inside and prevent contact between the absorbent material 4 and the dried cannabis or hemp flower 1B. The absorbent material 4 can be selected from a sponge, an absorbent cloth, or a cotton ball. In the preferred embodiment, the absorbent material 4 is a sponge in the shape of a sphere. The liquid can be selected from essential oils, terpenes, water, aroma, or flavors. In the preferred embodiment, the desired liquid is terpenes.

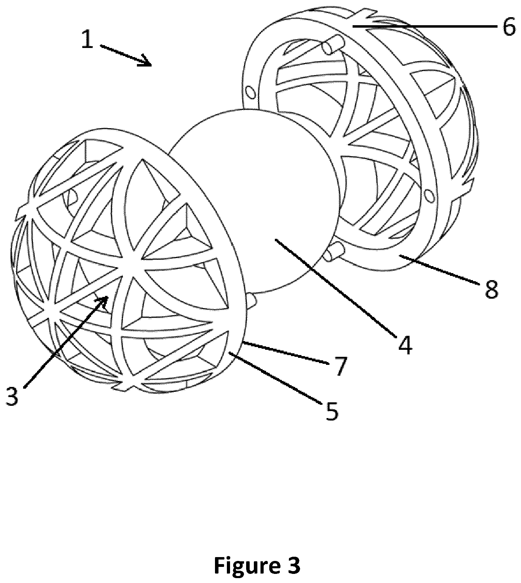

In the embodiment illustrated in Figures 3-6, the openwork shell 2 provides means for access to its interior and allowing easy entry and removal of the sponge 4. In the embodiment illustrated the means for access to the interior of the openwork shell is provided by the openwork shell 2 consisting of first 5 and second 6 parts wherein each part has a means for attachment to the other. In the embodiment shown the openwork shell 2 consists of two identical halves each having a means for attachment to the other allowing for a secure attachment between the halves and keeping the sponge 4 encased inside the openwork shell 2.

The embodiment of the openwork shell 2 for infusing dried cannabis or hemp flower 1B with an essential oil or one of the many different identified terpenes normally found in the cannabis or hemp plant shown in the Figures is molded from a thermoplastic material inert to the liquid, preferably essential oils. The openwork shell 2 is sized appropriately and has a shell thickness sufficient to contain an absorbent material 4 to fit comfortably and loosely inside the openwork shell 2. The openwork shell 2 illustrated has first 5 and second 6 parts. The first part 5 of the openwork shell 2 has a first rim surface 7 and means for attachment to a second rim surface 8 on the second part 6 in order to create a secure connection between the first 5 and second 6 parts of the openwork shell 20. In the embodiment illustrated, the means for attachment is a pin and hole system.

While the principles of the invention have been shown and described in connection with specific embodiments, it is to be understood that such embodiments are by way of example and are not limiting as is evident from the foregoing description, certain aspects of the present invention are not limited by the particular details illustrated in the drawings. Other modifications and applications, or equivalents, will occur to those skilled in the art. The terms "having", "comprising" and "including" and similar terms as used in the foregoing specification are used in the sense of "optional" or "may include" and not as "required". Many changes, modifications, variations and other uses and applications of the present construction will, however, become apparent to those skilled in the art after considering the specification and attached drawings. All such changes, modifications, variations and other uses and applications which do not depart from the spirit and scope of the invention are deemed to be covered by the invention which is limited only by the claims that follow. The scope of the disclosure is not intended to be limited to the embodiments shown herein, but is to be accorded the full scope consistent with the claims, wherein reference to an element in the singular is not intended to mean "one and only one" unless specifically so stated, but rather one or more. All structural and functional equivalents to the elements of the embodiment described throughout the disclosure that are known or later come to be known to those of ordinary skill in the art are expressly incorporated herein by reference and intended to be encompassed by the claims.

## Claims

1. A method for infusing dried cannabis or hemp flower with a liquid, selected from the group consisting of essential oils, terpenes, water, aroma, or flavors, to improve humidity, taste and effect, the method comprising placing a perforated casing containing an absorbent material with a selected essential oil added to the absorbent material into a container, adding dried cannabis or hemp flower and closing the container.

2. The method according to claim 1, wherein the perforated casing is an openwork shell.

3. The method according to claim 1 or 2, wherein the liquid is applied to the absorbent material before the absorbent material is placed into the casing.

4. The method according to claim 1 or 2, wherein the liquid is applied to the absorbent material after it is encased within the casing

5. The method according to any one of claims 1 to 4, wherein the liquid has been applied to the absorbent material using a liquid applicator means.

6. The method according to claim 5, wherein the liquid applicator means is a syringe.

7. The method according to claim 3, wherein the liquid has been applied to the absorbent material by soaking the absorbent material in the liquid, removing the absorbent material from the liquid, squeezing the excess liquid from the absorbent material, and letting the absorbent material with the liquid applied sit for a sufficient amount of time to absorb the liquid and fully saturate.

8. The method according to any one of claims 1 to 7, wherein the container holds dried cannabis or hemp flower.

9. The method according to any one of claims 1 to 7, wherein the apparatus can be sequentially used to infuse a plurality of batches of dried cannabis or hemp flower.

10. The method according to any one of claims 1 to 9, wherein the absorbent material is not over saturated by the liquid.

11. An apparatus to be used for infusing dried cannabis or hemp flower with a liquid, selected from the group consisting of essential oils, terpenes, water, aroma, or flavors, comprising a casing formed of a material inert or impervious to the liquid and having one or more apertures and an absorbent material contained within the casing; the absorbent material capable of retaining and diffusing the liquid applied to the absorbent material wherein the casing is an openwork shell having adequate thickness to encase the absorbent material inside and prevent contact between the absorbent material and the dried cannabis or hemp flower and wherein the openwork shell has a means for access to its interior allowing easy entry and removal of the absorbent material.

12. The apparatus according to claim 11, wherein there is sufficient aperture area on the openwork shell for the dried cannabis or hemp flower to be sufficiently infused and wherein the apertures are sized and shaped so as to not allow direct contact between the absorbent material and the dried cannabis or hemp flower and so as to allow the dried cannabis or hemp flower to be sufficiently infused.

13. The apparatus according to any one of claims 11 to 12, wherein the absorbent material is selected from one or more of a group consisting of a sponge, an absorbent cloth, or a cotton ball.

14. The apparatus according to any one of claims 11 to 13, wherein the liquid is an essential oil containing one or more terpenes.

15. The apparatus according to any one of claims 11 to 14, wherein the openwork shell encasing the absorbent material with the liquid applied is enclosed within a container, wherein the container has a means for separating the outside environment from a controlled environment inside the container.
